# EUROPEAN PATENT SPECIFICATION

(11) **EP 2 756 766 B1**
(45) Date of publication and mention of the grant of the patent: **11.01.2017**
(21) Application number: 13382462.3
(22) Date of filing: 15.11.2013
(51) Int. Cl.: A23L 3/3463, A23B 4/027, A23L 27/00

(54) **Brine and method for the manufacture thereof**
Lake und Verfahren zur Herstellung davon
Saumure et son procédé de sa fabrication

(30) Priority: 17.01.2013 ES 201330047
(43) Date of publication of application: 23.07.2014
(73) Proprietor: Crespo Cardona, Jorge Diaz, 03003 Alicante (ES)
(72) Inventor: Diaz Crespo Cardona, Carlos Pascual, 03003 Alicante (ES)
(74) Representative: Carlos Hernando, Borja

(56) References cited:
- WO-A1-2012/027901
- CN-B- 102 070 271
- US-A- 5 262 186
- US-A1- 2006 105 082
- US-B1- 6 875 457
- N E ET AL: "CONCENTRATED BRINE PRODUCTION FROM SEA WATER BY ELECTRODIALYSIS USING ION EXCHANGE MEMBRANES", , 2 April 1969 (1969-04-02), pages 159-165, XP055132879, Retrieved from the Internet: URL:http://pubs.acs.org/doi/pdf/10.1021/i2 60030a003 [retrieved on 2014-08-01]
- REDONDO J ET AL: "Boron removal from seawater using FILMTEC<TM> high rejection SWRO membranes", DESALINATION, ELSEVIER, AMSTERDAM, NL, vol. 156, no. 1-3, 1 August 2003 (2003-08-01), pages 229-238, XP004452696, ISSN: 0011-9164, DOI: 10.1016/S0011-9164(03)00345-X
- M TANIGUCHI: "Boron reduction performance of reverse osmosis seawater desalination process", JOURNAL OF MEMBRANE SCIENCE, vol. 183, no. 2, 1 March 2001 (2001-03-01) , pages 259-267, XP055132966, ISSN: 0376-7388, DOI: 10.1016/S0376-7388(00)00596-2
- KABAY N ET AL: "Boron in seawater and methods for its separation - A review", DESALINATION, ELSEVIER, AMSTERDAM, NL, vol. 261, no. 3, 31 October 2010 (2010-10-31), pages 212-217, XP027252741, ISSN: 0011-9164 [retrieved on 2010-06-12]
- Anonymous: "VITALIZAN EL AGUA DEL MAR PARA CONSUMO HUMANO", Discovery DSalud, 1 June 2005 (2005-06-01), pages 1-3, XP055132868, Retrieved from the Internet: URL:http://www.dsalud.com/index.php?pagina =articulo&c=521 [retrieved on 2014-08-01]
- Louis J Ronsivalli ET AL: "Low Temperature Preservation of Seafoods: A Review", , 1 April 1981 (1981-04-01), XP055133105, Retrieved from the Internet: URL:http://spo.nmfs.noaa.gov/mfr434/mfr434 1.pdf [retrieved on 2014-08-04]
- Anonymous: "Grander Belebtes Wasser", , 10 October 2012 (2012-10-10), XP002728152, Retrieved from the Internet: URL:https://web.archive.org/web/2012111006 3936/http://grander.com/de/grander-effekt/ der-nutzen [retrieved on 2014-08-04]

## Description

### Technical Field of the Invention

The present invention relates to a method for manufacturing a brine with improved features for use in food as a condiment and for the preservation and treatment of fish and shellfish, improving the organoleptic and nutritional properties of the foods treated and/or cooked with it. The brine with improved features obtained according to the method of the present invention can furthermore be used as raw material for manufacturing intensely hydrating and mineralizing isotonic beverages for human consumption, as well as any food product requiring water in the manufacturing process, improving the nutritional properties thereof. It can also be used as raw material for manufacturing products with a high nutritional value in agriculture and stock raising, such as feed and mineral supplements for stock raising.

Furthermore, the brine of the present invention can be used in processes of the food industry or any type of industry that requires using water with a high salt, nutrient and alkali content and with certain preserving ability and an ability to inactivate bacteria and microorganisms in general.

### Background of the Invention

There is currently a plurality of mineral-based products extracted from seawater that can be used for human consumption and in agriculture even though they all have some limitation restricting their use.

Among others, Spanish patent document ES2343777 describes a method for collecting and packaging seawater and its pharmaceutical use to complement mineral deficiency in humans. The method described in this patent comprises, among others, a phase for the microfiltration and mixture of the extracted seawater with bacteriologically pure and naturally sterile non-mineral and non-medicinal water, although said method does not incorporate a deboronation and revitalization phase, so the product obtained using said method necessarily contains a boron concentration substantially similar to that present in seawater.

Other documents from the state of the art, such as US 7,442,309, relate to methods for treating salt water including a step to remove the boron content therefrom. Said methods nevertheless include a reverse osmosis step, whereby although boron is effectively removed, other minerals and salts present in seawater are also removed, which inevitably leads to the obtained product lacking the combination of minerals present in seawater in its natural state.

US2012/0160753 relates to a seawater desalination plant using a nanofiltration step to remove boron. Nevertheless, the method used incorporates a reverse osmosis step which likewise removes other minerals and salts present in seawater in its natural state.

N E ET AL: "CONCENTRATED BRINE PRODUCTION FROM SEAWATER BY ELECTRODIALYSIS USING ION EXCHANGE MEMBRANES", 2 April 1969, pp 159-165, describes a brine composition free of substances and macromolecules of a size greater than 10 - 100 microns, which is obtained by a method comprising electrodialysis using ion exchange membranes which contemplates filtering through a synthetic fiber cloth, wherein such filtering process is capable of removing substances of a size greater than 10 - 100 microns.

US 2006/105082 discloses a process for the recovery of useful products, including fertilizers and nutritional supplements, from the organic matter and minerals contained in seawater and other brines. However the process herein described does not comprise two microfiltration steps, deboronation and a vitalization treatment.

Based on the foregoing, the object of the present invention is to provide a novel method for obtaining a brine with improved features, free of organic matter, macromolecules and bacteria, which maintains the mineral load and composition of natural seawater, with the exception of boron, has higher alkalinity and an improved molecular structure. Significant industrial application advantages which are better and more unique than those of common drinking water, water with dissolved sea salts, the use of non-dissolved common salt or sea salt or the use of untreated seawater itself, derive from these improved product features.

### Description of the Invention

The present invention relates to a method for obtaining a brine with improved features which maintains the natural mineral structure and composition of the seawater, i.e., seawater free of substances that are harmful for human consumption, among others, boron, comprising the following phases or steps:
a) Seawater extraction
b) Purity analysis
c) First microfiltration
d) Deboronation treatment
e) Increase in pH
f) Vitalization treatment
g) Second microfiltration
h) Microbiological analysis
i) Packaging

A brine that can be used directly for the treatment and preservation of food products, among others, fish and shellfish, as a condiment and/or that can be used as a raw material base for the preparation of products for human consumption, in agriculture and stock raising, is obtained with this method.

Regarding the use of the brine of the present invention for the treatment and preservation of food products, the present invention contemplates providing liquid products the application of which on the food to be treated takes place, for example, through spraying, or preparing synthetic supports of the type consisting of paper, cloth or towel impregnated with the brine of the present invention, which enable packaging or covering the food to be treated.

With respect to the possibility of using the brine of the present invention for the preparation of products for agricultural use and stock raising, the incorporation thereof in its original concentration, i.e., directly, or in its diluted form to feeds and mineral supplements for livestock is contemplated.

An object of the present invention is therefore a method for obtaining a brine characterized in that it presents the mineral composition of the seawater in its natural state, with a boron content less than 1 mg/l, free of organic components, bacteria and macromolecules of a size greater than 0.1 µ, a pH between 8.6 - 8.8, is vitalized, and presents the angle in the molecular structure of the bonds of the hydrogen atoms with the oxygen atom of highly pure natural spring water

Another object of the present invention is therefore an improved brine for use in food having the mineral composition of the seawater in its natural state, with a boron content less than 1 mg/l, free of organic components, bacteria and macromolecules of a size greater than 0.1 µ, a pH between 8.6 - 8.8, is vitalized, and presents the angle in the molecular structure of the bonds of the hydrogen atoms with the oxygen atom of highly pure natural spring water,obtainable through the method of the present invention.

Food products, cooking products and generally products for human as well as animal consumption or products for being applied in agriculture and stock raising containing the brine of the present invention as an ingredient are also an object of the present invention.

Synthetic supports of the type consisting of paper, cloth or towel impregnated with or containing the brine of the present invention for use in food preservation are also an object of the present invention.

### Detailed Description of the Invention

The object of this invention is primarily the development of a technology that allows obtaining a brine obtained with improved features from seawater, having the following technical properties: the same mineral composition as the original seawater, with the exception of boron the content of which is less than 1 mg/L, being free of organic components, bacteria and macromolecules of a size greater than 0.1 µ and with a pH between 8.6 - 8.8, due to the addition of bicarbonates and carbonates.

Another essential feature of the brine of the present invention is that it is vitalized, i.e., the molecular structure of water is improved, by modifying the angle of the bonds of the hydrogen atoms with the oxygen atom as it is found in highly pure natural spring water.

Therefore, the brine of the present invention is a unique product combining the original properties of seawater primarily derived from its complete mineral composition, in an organic and bioavailable form, the properties derived from its molecular restructuring, as a result of the method used for obtaining same, is alkaline with a pH between 8.6 and 8.8, and it further has all the health guarantees necessary for complete food safety.

The brine of the present invention can be used directly for the preservation and treatment of fish and shellfish and as a condiment. Its advantages in this application are greater than those obtained through the use of water with dissolved sea salts, non-dissolved common salt or sea salt or the use of untreated seawater itself.

Seawater contains virtually everything that exists on earth, all the elements from the periodic table, and in proportions that are very similar to those in blood plasma. Furthermore, the sea is a solution of electrolytes, electrically-charged atoms called ions, with an incalculable biological value. As a result of this feature, these elements can cross the skin and the cell membranes, being readily assimilable by cells. Fully reconstructing seawater by means of salts is not possible; important properties are lost with regard to the variety of elements present and the bioavailability of such elements. The brine of the present invention furthermore has a greater solvent effect, better cell hydration capacity and a better capacity to inactivate microorganisms that seawater itself. Furthermore, since it is somewhat more alkaline than seawater in its natural state, it has a higher effect of inactivating bacteria and microorganisms and a higher capacity to neutralize acids produced in the tissues of fish and shellfish once they are extracted from the sea, during the natural decomposition process, so it is more effective in preserving these products.

The use of the brine of the present invention in the preservation of sea products allows preserving the freshness of foods for a longer time, avoiding the use of chemicals or aggressive physical or mechanical processes that destroy the molecular structure of foods and entail a high economic cost.

Specifically, the main advantages of the use of the brine of the present invention for the indicated purposes are as follows:
- Reduction of water loss in the treated products during preservation, achieving better hydration in tissues and cells of sea products.
- Reduction of the loss of nutrients in the treated products during preservation, maintaining the nutritional value thereof.
- Delay in the treated product decomposition process, minimizing microorganism proliferation and acid production relating to the decomposition process, thus prolonging the time for optimal consumption.

- Reduction of contamination by pathogens such as *E.coli, Clostridium, Listeria,* etc., characteristic of other sea product treatment processes.
- Maintenance of the organoleptic properties of fish and shellfish, texture, appearance and natural color for a longer time period.
- Reduction of the "bad smell" associated with the production of lactic acid produced during the natural decomposition process of fish and shellfish.

Ultimately, the use of the brine of the present invention for the treatment and preservation of fish and shellfish increases the quality, consumption safety and cost-effectiveness thereof.

As described above, the brine of the present invention can also be used directly to wash and thaw foods in general, and it can also be used as a condiment, providing a large amount of essential nutrients in a natural, organic and bioavailable form.

The use of the brine of the present invention in the preparation of foods helps keep the body in a slightly alkaline, optimal pH range, provides a large amount of organic nutrients readily assimilable by the cells, in addition to providing nutrition and hydration to said cells, favoring the self-repair capacity thereof, and re-balancing metabolic processes.

Among others, the health effects provided in that field of application are as follows:
- Better cell nutrition, providing a large amount of essential nutrients in an organic and readily assimilable form.
- It favors maintenance of the body's acid-alkaline balance.
- It favors light digestions, reducing hyperacidity.
- It favors cell hydration, promoting cell rejuvenation.
- It favors reducing common salt consumption.
- It stimulates kidney function, favoring liquid elimination.
- It controls hypothyroidism and helps to maintain optimal weight.
- It contains trace elements that stimulate and regulate the immune system.
- It optimizes muscle recovery after physical exercise.

Additionally, the use of the brine of the present invention as a condiment brings out the original flavors and textures of the foods in which it has been incorporated and offers freshness.

Furthermore, the brine of the present invention can be used as raw material or active ingredient in manufacturing food products with beneficial health effects, such as isotonic or intensely hydrating beverages, or for improving the nutrient status of industrial foods and products such as bread, doughnuts, pastries, pizzas, pasta, canned foods, fried foods, juices, beer. Depending on the use, the suitable proportion of brine diluted with drinking water is added to obtain the suitable amount of salt.

Furthermore, the brine of the present invention can be used in processes of the food industry or any type of industry that requires the use of water with a high salt, nutrient and alkali content and with certain preserving ability and an ability to inactivate bacteria and microorganisms in general. For example, the brine of the present invention can be used in washing fruits and vegetables; producing canned foods, pickled foods, salted foods; cleaning, preserving and thawing products from the sea, as well as applications related to health and beauty treatments, marine hydrotherapy, thalassotherapy, spas, pools, maintaining and conditioning aquariums, fish tanks or any recreation of marine environments with a biological value.

The present invention also relates to the method for obtaining the brine with improved features described above, maintaining the natural mineral structure and composition of seawater, free of substances that are harmful for human consumption, among others, boron, said method characterized in that it comprises the following phases or steps:
a) Seawater extraction,
b) Purity analysis,
c) First microfiltration,
d) Deboronation treatment,
e) Increase in pH,
f) Vitalization treatment,
g) Second microfiltration,
h) Microbiological analysis,
i) Packaging.

### a) Seawater extraction:

Seawater extraction is performed from fixed points, pipelines or moving points, tankers duly equipped with extraction pumps and tanks approved for use in food. The points of extraction or seawater source where the method for obtaining the brine of the present invention begins are located in areas under special environmental protection, such as for example, The Nature Reserve of Tabarca, of enormous value due to its biological diversity in phanerogam beds and the fauna associated with them, for the purpose of obtaining water with the highest possible quality.

Before extraction, determinations of at least salinity, turbidity, chlorophyll (phytoplankton) and dissolved oxygen are taken by means of a multiparametric probe capable of measuring the entire water column in real time. The point and the depth where these parameters are optimal are selected based on the results. Locations with a salinity that is never less than 35 g/l, small quantity of phytoplankton, low turbidity (never greater than 1 NTU) and with normal oxygenation (there are no hypoxia processes, never below 95% oxygen saturation) are chosen.

### b) Purity analysis:

The seawater extracted from the fixed or moving point, such as with the tanker, for example, is transferred with the aid of pumps suitable for this purpose to polyethylene tanks approved for use in food located in the processing plant itself. These tanks have a 26,000 liter capacity, and this volume is what is obtained in each extraction operation from the moving or fixed point, so the manufacturing batches are of 26 cubic meters. Each of these batches is analyzed and validated according to the results as a prior requirement to enable using it in manufacturing the brine of the present invention.

For that purpose, standard physicochemical and microbiological analyses known by the person skilled in the art are performed following normalized methods (see the attached table) to verify that it is free of pollutants and complies with the parameters established by specific regulations, i.e., Royal Decree 1424/1983 of April 27, which approves the Technical-Sanitary Regulation for obtaining, circulating and selling edible salt and brines, and furthermore, for want of a specific regulation on the treatment and use of seawater, it must also comply with the recommendation of the EFSA (European Food Safety Authority) in the EFSA Scientific Evaluation (EFSA Journal 2012;10(3):26139), which includes some of the parameters comprised in Royal Decree 140/2003, limiting the concentration of possible chemical and biological pollutants. These determinations and the criteria followed for validating extracted seawater will be broadened and/or modified provided it is necessary in order to comply with the regulations in force at all times and with the recommendations established by renowned organizations such as EFSA.

| Parameter | Testing method/Protocol |
|---|---|
| Organoleptic analysis | PNTM3087 (UNE-EN 1622) |
| Conductivity | B.O.E. O. 1.7.1987 |
| pH | B.O.E. O.15.9.1985 |
| Turbidity | ISO 7027 |
| Sulfates, oxidizability, nitrites | B.O.E. O.1.7.1987 |
| Nitrates | B.O.E. O. 1.7.1998 |
| Aluminum, boron, cadmium, copper, chromium, iron, manganese, nickel, sodium | PNTA0141 (ICP-AES) |
| Arsenic, mercury, lead, selenium, antimony | PNTQ1032/AAS/GF) |
| Cyanides | APHA 4500-CN E |
| Chlorides | PNTA0078 |
| Aerobic microorganisms (22°C) | PNTM3000 |
| Total coliforms | PNTM3045 |
| *Clostridium perfringens* | PNTM3086 |
| *Escherichia coli* | EN ISO 9308 |
| *Enterococcus* | EN ISO 7899 |
| *Vibrio spp* | ISO/TS 21872:2007 |
| Halogenated and non-halogenated solvents, aromatic polycyclic hydrocarbons, pesticides | GC/MS |

Maximum levels of chemical pollutants are established in Directive 98/83/EC on the quality of water intended for human consumption, and additionally the maximum limits of barium and manganese are established in Directive 2003/04/EC, shown in Appendix 1 of the mentioned EFSA Scientific Evaluation.

Based on these parameters contemplated in EFSA's report, the boron content is the only parameter with which natural extracted seawater does not comply. Seawater contains about 5 mg/l of boron, whereas the limit recommended by EFSA is 1 mg/l. As a result, the process for obtaining the brine of the present invention contemplates a deboronation step or treatment which will be explained in detail below.

### c) First microfiltration:

According to the regulations in force and EFSA's recommendation for the use of brines in the food field, organic components such as phytoplankton, zooplankton and bacteria, must be removed from seawater. Bacteria must be removed with a method of proven efficacy, such as microfiltration with a pore size of 0.22 microns, approved by the European Pharmacopoeia as a safe sterilization method.

For that purpose the extracted seawater must be subjected to a microfiltration that allows removing the organic and macromolecular components from the water without modifying the original mineral composition thereof, i.e., neither the concentration nor the form of the mineral elements in seawater are affected in any way by this water treatment.

Microfiltration is performed in two steps: in the first step, the water passes through a membrane filter with a pore size of 1 µ, and in the second step it passes through a membrane filter with a pore size of 0.22 µ, both steps being capable of removing from the seawater substantially all the components larger than 0.22 µ without compromising the mineral composition and concentration of the original seawater.

The filters used are pleated hydrophilic Durapore CBR cartridge filters with a PVDF membrane and extractable polypropylene components providing high flow rates and low outputs with broad chemical compatibility.

In reference to bacterial retention, the tests conducted by Millipore, the manufacturer, confirm that Hydrophilic Durapore CBR membranes retain 107 CFU per cm² in tests with *Brevundimonas diminuta* and comply with the sterilizing grade performance as it is defined by ASTM methodology.

By means of a prior buffer tank and a system of electrovalves a flow rate with constant pressures is maintained to assure good filtration. At the end of each day, the filters are cleaned and disinfected with disinfectant approved for use in food and with decalcified hot water.

### d) Deboronation treatment:

Boron in the seawater in its natural state has a mean concentration of about 5 mg/L. However and according to the EFSA's (European Food Safety Authority) recommendations, the concentration of this element in brines originating from seawater for food consumption must be at a level of less than 1 mg/L.

For this reason the method for obtaining brine according to the present invention must necessarily incorporate a deboronation step capable of reducing said boron concentration levels until reaching a concentration of less than 1 mg/L.

The most important and essential aspect of this step is, however, to perform the selective removal of boron, thus maintaining the remaining mineral concentration and composition of seawater in its natural state.

To that end a selective ion exchange demineralization system which allows separating the boron from the water without affecting either the concentration or the form of the remaining mineral elements present in the water by means of using a specific solid matrix (resin), is used.

The type of specific resins to be used are conventionally strong base anion resins (tertiary amino functional group bound to a polyamide base matrix), such as Amberlite PWA 10 with very high selectivity for boron and approved for use in water for consumption or water that come into contact with foods.

The production cycle of this deboronation system comprises the production time plus the time for regenerating the resin by means of washing with an acid (for example H₂SO₄) and subsequently neutralizing with a base (for example NAOH). For a production flow rate of 10 m³/h, the total production cycle is 29 hours long, 26 hours of which being the time actually invested in producing deboronated water and the remaining 3 hours being the time invested in regenerating resins. The resin used in each production cycle has an exchange capacity of 1.87 g of boron per liter of resin, whereas the rejection rate is of 1.7% of the total water processed, which is 255 m³.

The rejection water rich in boron, sodium and sulfates is managed as a hazardous toxic waste and assigned to an authorized waste treatment manager as established in the regulations in force in relation to hazardous waste management.

### e) Increase in pH:

The pH of the brine of the present invention increases up to a value greater than 8.2, preferably between 8.6 - 8.8. An additive for use in food called a pH buffer made up of a mixture of bicarbonate/carbonate at a ratio of 5:1 is used to increase the pH.

The bicarbonate/carbonate ratio in seawater in its natural state is 9:1 and this ratio is what primarily determines the pH value of 8.2 present in the seawater. By adding this additive at a smaller ratio, i.e., a higher proportion of carbonate, pH of the water increases. It is important to add the bicarbonate and the carbonate together so that the carbonate does not react with Ca²⁺ present and precipitate in the form of calcium carbonate.

This pH buffer is applied in an intermediate tank under constant stirring and in several steps until achieving the desired pH value, i.e., greater than 8.2, preferably between 8.6 - 8.8, without allowing precipitates to form.

To that end is used an automatic additive metering device of the type controlled by a control center which receives a continuous reading from a pH probe, which analyzes the water based on recirculation in the mentioned tank.

When the computer system detects that the batch being produced reaches the correct pH value, it sends the water to the next step of the process.

### f) Vitalization treatment:

The properties of the seawater are considerably improved in this treatment step in a natural manner, without adding or removing any component or element to/from said seawater. Vitalization treatment does not act on the composition of the water but rather on its molecular structure.

Japanese scientist Masaru Emoto has demonstrated with a number of experiments gathered in thousands of microphotographs that water has the capacity to store and transmit information by alternating its molecular structure as a reaction to any positive or negative message it receives. Over 20 years ago, Johann Grander came up with a technology that allows vitalizing water by returning its natural potential, original molecular structure and furthermore making it more resistant to contamination by pathogenic microorganisms. In other words, his apparatuses successfully create what is called an "immune system for water", making it stronger and giving back its capacity to be purified, in order to preserve it in a vitalized and structured form for a longer period of time.

In the process for obtaining vitalized water according to the present invention "Grander" technology is applied, whereby the seawater obtained in the preceding step is vitalized by using the commercially available Grander method.

Grander technology is based on the capacity of water to gather information and transmit it to other water by bioresonance.

The Grander devices used in the process for producing the brine of the present invention contain water carrying Grander information. As the brine water passes through these devices, it "copies" this information, becoming revitalized, enhancing its regeneration capacity and returning to a state of high molecular order.

The vitalization process modifies the structure of water molecules, changing the angle formed by the bonds of the two hydrogen atoms with respect to the oxygen. The water molecules thus associate with one another in perfect geometrical forms.

The features given to the water through this vitalization treatment phase are a restoration of the original strength of the water, an enhancement of its self-regeneration and self-purification capacity, a reduction of radioactivity and an increase in its resistance against negative external influences.

The benefits of applying Grander technology to the process for manufacturing the brine of the present invention are:
- In fish and shellfish preservation and treatment. The vitalized brine increases the preservation time of these perishable products, considerably reducing the growth of pathogenic microorganisms.
- It increases the capacity of keeping fish and shellfish hydrated for a longer period of time. The vitalized brine has a greater capacity of penetrating cells and tissues.

Experiments that have been carried out demonstrate that the effectiveness in preserving fish increases considerably if the fish has been previously treated with seawater or brine previously treated with Grander technology.

In conclusion, using seawater vitalized with Grander technology is to add beneficial effects to fish and shellfish preservation and treatment. The natural disinfecting power of seawater, its capacity to keep fish and shellfish hydrated, maintaining their organoleptic properties, texture, and color, in addition to the capacity of water vitalized with Grander technology to automatically clean itself and control microorganism proliferation, its energizing and hydrating capacity, in turn, make this brine a highly efficient ally in fish and shellfish preservation for the fishing industry in general.
- Regarding the use of brine in cooking to prepare foods, the process of vitalization with Grander methodology also provides greater food safety due to the protection conferred to foods against the microbial growth of bacteria as worrisome in the food field as *Escherichia Coli, Salmonella, Enterobacteriaceae, Listeria,* etc.

Furthermore, Grander technology applied to seawater enhances its hydrating effect, nutrient status and energizing capacity, and generally its capacity to promote health.
- In manufacturing isotonic beverages, Grander vitalization technology clearly enhances the effects on health to be offered with this product. Molecularly structured water has a greater solvent effect and greater cell hydration capacity, in addition to its energizing effect. Therefore, the body's assimilation of the nutrients and electrolytes supplied by seawater is greater favored, enhancing its recovery effect after straining and sweating while practicing sports.

### g) Second microfiltration:

Before packaging the end product, the seawater obtained in the preceding step is subjected to final 0.1 µ microfiltration in membrane filters with a pore size of 0.1 µ. This second microfiltration is also performed by means of using Hydrophilic Durapore CBR filters, and it additionally allows assuring complete sterilization (removal of bacteria), removing a large amount of viruses, particularly viruses associated with bacteria.

Any bacterium of a size greater than 0.1 µ that may have contaminated the product during processing is removed with this microfiltration, assuring end product food safety.

### h) Microbiological analysis:

Once the water is processed and before being used for packaging or bulk sale, it is re-analyzed to check process reliability and assure the complete absence of bacteria in each of the batches produced. To that end, once 26 m³ of each batch is processed following all the steps described above, they are stored in tanks of that capacity, a minimum storage period of 48 hours starting at that time, and this is the time used to take microbiological determinations that are needed to validate the quality of the water and market it.

If a colony forming unit is obtained in any of the analyses performed, the batch is discarded and the causes of the contamination are studied, checking the production process and the operation of the equipment involved in said process.

The microbiological determinations to be taken are: *mesophilic aerobic bacteria at 22°C* (PNTM3000 method), *total coliforms* (PNTM3045 method), *Escherichia coli* (EN ISO 9308 method), *Enterococcus* (EN ISO 7899), *Clostridium perfringens* (PNTM3086), *Vibrio spp (ISO*/*TS 21872:2007).*

### i) Packaging

After at least 48 hours have passed and the microbiological quality of the brine has been validated with the analytical confirmation of the absence of bacteria, the product can be packaged or loaded into vats for bulk transport to later be sold.

With respect to the products incorporating the brine of the present invention, as has been described in preceding sections, the brine of the present invention can be incorporated directly or after being diluted to products indicated for human, animal and agricultural use or consumption.

In all these cases, the brine of the present invention is added in liquid form and without prior dilution or treatment to the usual ingredients contained in such products, in amounts ranging between 1% and 99% by weight with respect to the total weight of the composition, preferably between 70% and 95% by weight with respect to the total weight.

The following examples are described below to illustrate the present invention.

### EXAMPLES:

### Example 1:

Study of the effect of the brine of the invention on the dehydration of fresh fish and shellfish in the first 72 hours after being extracted from the sea.

The product tested in this test was obtained following the method described in the general description.

### Objective of the study:

To comparatively evaluate the effect that the brine of the present invention and mains drinking water have on the preservation of fish and shellfish, the dehydration thereof and the possible weight variation in the first 72 hours after being extracted from the sea.

### Description of the test:

The study conditions were as similar as possible to those which are found in large supermarket fish shops. The conditions were as follows:
The fish and shellfish were representative and diverse, "wild", in no case originating from the aquaculture industry or the frozen food industry, and therefore recently fished. It was obtained directly from the fish market, immediately after being extracted from the sea.

The study was conducted as a comparative experiment on 5 different types of sea products:
- small blue fish: anchovy (*Engraulis encrasicolus*)
- medium- or large-sized white fish: hake (*Merluccius merluccius*)
- cephalopods: octopus (*Octopus vulgaris*)
- bivalve molluscs: mussel (*Mytilus edulis*)
- crustaceans: Mediterranean shrimp (*Aristeus antennatus*)

The amounts of each of them were greater than 1,000 g, the necessary amounts for conducting the tests. The results on the comparative dehydration were obtained for each of these 5 types of sea products. In no case were the entrails or any part whatsoever removed from the fish and shellfish. The products were preserved whole for the duration of the study.

Mains drinking water and the brine of the present invention were used thinly sprayed on the fish and shellfish using an automatic spray system and previously tested.

The fish was placed on plastic trays with flake ice similarly to how fish is arranged in supermarket fish shops. Each type to be studied was placed on a tray separately.

The fish and shellfish were visibly placed on a bed of ice in each of the trays, as they are arranged in displays in the mentioned fish shops.

A cold room was used to store the fish and shellfish overnight at a temperature of 1-3°C.

### Method:

The test lasted for a total of 72 hours. The start time was 9:30 am on the first day and the fish was acquired at the fish market the day before or on the same day a few hours before, recently extracted from the sea. The end time of the test was 10:00 am on day 4, after exactly 72 hours. Therefore, on day 4 the fish was simply taken out of the cold room and a final weighing was performed.

The fish and shellfish were preserved for 72 hours during which the test lasted in two different ways.
- During business hours from 9 am to 9 pm, they were preserved in plastic trays with a bed of flake ice, at room temperature, outside the cold room. This ice was replaced throughout the day as needed to maintain the correct preservation conditions.
- Overnight, from 9 pm to 9 am the next day, outside the business hours of a standard supermarket, the fish and shellfish were preserved in a cold room at a temperature of 1-3°C.

The types of water were sprayed on the trays of fish and shellfish every 5 minutes throughout all business hours, from 9 am to 9 pm, on the three days during which the test lasted. For these spray applications to be similar to those used by automatic sprinklers in supermarkets, they were preformed as follows:
- The sprayed water fell on the fish slowly, from a zenith position, in the form of rain, preventing the water from impacting the surface of the fish and shellfish at a high speed. To that end, the nozzles of the spraying system were placed at a distance of 70 centimeters from the fish and on the vertical of the tray.
- Periodic spraying every 5 minutes completely wetting the entire surface of the fish and shellfish.
- Each spray application in the automatic sprinklers lasted for 1.5 seconds.
Therefore, the fish was sprinkled with a gentle fall of water during this time.

Each of the control units was weighed every hour during the time in which they were placed on the trays with ice, from 9:00 am to 9:00 pm, business hours.

A first weighing was taken at the beginning of the day, at 10:00 am, and after that every hour until 9:00 pm. The fish was preserved in the cold room at night and were not weighed.

The test was conducted using 3 groups. The 5 types of sea products were represented in each of these groups.
- Group 0. Non-sprayed control group.
- Group 1. Group sprayed with water from the Mediterranean Sea.
- Group 2. Group sprayed with drinking water from the tap (mains water).

Each of the control groups comprised the necessary number of test units according to the type of fish to enable subjecting the results to suitable statistical treatment.

### Data acquisition and results:

Weight control. Results on the evolution over time of the net weight of each of the types of fish and shellfish in the 3 groups were obtained. Comparisons between the types of sea products in each group and comparisons between the 3 groups were made with this data.

In parallel, observations were made by visual inspection of subjective aspects such as the general appearance, texture, consistency of the tissues and the smell of each type of sea product throughout the experiment.

These observations were made every 6 hours, 3 observations a day, one at 9:00 am, another at 3:00 pm and the last one of every day at 9:00 pm. The differences obtained in each of these aspects were established in a comparative manner between the control groups for each of the types of fish and shellfish.

### Results and discussion:

### Weight variation:

Efficiency of the use of the brine of the present invention versus mains water, regarding its hydrating capacity reflected in the weight variations obtained:

| Item | Difference in % |
|---|---|
| White fish: hake | +5.70 |
| Cephalopods: octopus | +7.80 |
| Bivalve molluscs: mussel | +3.40 |
| Crustaceans: shrimp | +3.80 |
| Blue fish: anchovy | +6.60 |

The positive (+) result shows a differential that favors the brine of the present invention.

### Sensorial analysis:

An evaluation of the quality of the fresh fish was evaluated using the "quality index method" or QIM.

The QIM uses a practical rating system in which the fish is inspected and the corresponding demerits are recorded. The scores recorded in each feature are added up to give a total sensorial score, the so-called quality index. The QIM assigns a score of zero to very fresh fish; therefore, the higher the score the greater the deterioration of the fish (scale of 0 to 20 points). Evaluated quality parameters:

| Quality parameter | Feature |
|---|---|
| General appearance | Skin |
| | Blood stains |
| | Hardness |
| | Belly |
| | Smell |
| Eyes | Clarity |
| | Shape |
| Gills, where appropriate | Color |
| | Smell |

| Item | Time (hours) | QIM | | |
|---|---|---|---|---|
| | | Control | Mediterranean | Mains water |
| White fish: hake | 24 | 1 | 1 | 1 |
| | 48 | 6 | 2 | 7 |
| | 72 | 12 | 6 | 10 |
| Cephalopods: octopus | 24 | 1 | 1 | 1 |
| | 48 | 7 | 3 | 6 |
| | 72 | 9 | 8 | 9 |
| Bivalve molluscs: mussel | 24 | 1 | 0 | 0 |
| | 48 | 1 | 1 | 3 |
| | 72 | 3 | 2 | 3 |
| Crustaceans: shrimp | 24 | 0 | 0 | 0 |
| | 48 | 3 | 3 | 4 |
| | 72 | 7 | 5 | 8 |
| Blue fish: anchovy | 24 | 0 | 0 | 0 |
| | 48 | 4 | 1 | 3 |
| | 72 | 10 | 6 | 9 |

### Conclusions:

Treatment by means of spraying the brine of the present invention generally translates into an increase in hydration and therefore in the weights of the different species of fish and shellfish used in this study.

The differences found between the weights of group 0 (non-sprayed fish) and group 2 (sprayed with mains water) with respect to group 1 (sprayed with the brine of the present invention) are statistically significant.

Treatment of the fish on display by means of spraying with the brine of the present invention maintains the organoleptic properties typical of the different species of fish studied for a longer period of time when compared with non-sprayed fish or fish sprayed with mains water.

Treatment with the brine of the present invention delays the onset of unpleasant smells in the fish, and once these smells have set in, it reduces the intensity thereof.

### Example 2:

The brine of the present invention can be used as raw material for preparing foods at the industrial level. The brine of the present invention is added mixed with drinking water or fresh water in the proportion required in each case for the purpose of obtaining the suitable amount of salt. A table with the approximate proportions to be used in industrial food preparation processes is provided below:

| Food | Brine of the invention (%) | Fresh water (%) |
|---|---|---|
| Bread and pastries | 25 | 75 |
| Pizza dough | 25 | 75 |
| Beer | 15 | 85 |
| Isotonic beverage | 40 | 60 |
| Juices | 20 | 80 |
| Fried foods | 20 | 80 |
| Canned foods | 60 | 40 |
| Pasta | 20 | 80 |

The conditions under which the brine of the present invention is added are those that are determined in each case by the actual process for producing the food.

In the field of home cooking and restaurant businesses, the brine of the present invention can be used to be added to the water for cooking foods while preparing many kinds of recipes. In this case, the addition thereof mixed with fresh water in the proportion provided in the following table for each type of dish is recommended:

| Recipe | Fresh water (%) | Brine of the invention (%) |
|---|---|---|
| Shellfish | 0 | 100 |
| Fish | 65 | 35 |
| Potatoes | 65 | 35 |
| Vegetables | 70 | 30 |
| Chicken | 75 | 25 |
| Pasta | 75 | 25 |
| Rice | 80 | 20 |
| Salads | 85 | 15 |

Nevertheless, the final proportion can vary with respect to the amount of salt according to the end consumer's tastes.

### Example 3: Toothpaste (not in the scope of the claims)

The toothpaste prepared with the brine of the present invention is completely natural, without artificial additives, and has a series of properties very beneficial for oral health. This toothpaste is antiseptic, detoxifying, disinfectant, epulotic, immunostimulatory and antibacterial. The natural essences of aromatic plants give it a very pleasant flavor and smell and leave a very fresh feeling in the mouth.

The ingredients (in % by weight with respect to the total weight) used for preparing said toothpaste are as follows:
50% brine of the present invention (not diluted, used directly)
1% xanthan plant gum
48% white clay, kaolin, suitable for internal use
0.6% natural essences of aromatic plants such as peppermint, thyme, rosemary, lemon
0.4% vegetable dye

### Method of manufacture:

White clay is dissolved in the brine of the present invention at a ratio of 5 g kaolin for every 10 ml of brine of the present invention.

Natural plant essences together with the vegetable dye are added. They are mixed until perfectly homogenized. Powder xanthan gum, which is used as a thickener, viscosity agent, stabilizer and suspension agent is added, improving the texture of the paste. A proportion of 1.0% is used.

### Example 4: Mouthwash (not in the scope of the claims)

The mouthwash prepared with the brine of the present invention is completely natural and is free of artificial coloring and preservatives, sweeteners and of course alcohol. It has excellent properties that help keep the mount in a perfect state of health. This mouthwash is antiseptic, disinfectant, detoxifying, epulotic, immunostimulatory and antibacterial. It contains natural essences of plants such as peppermint, mint, thyme, rosemary and lavender, and stevia, giving it a somewhat sweet and very refreshing flavor.

The ingredients used for preparing it are as follows:
90% brine of the present invention (not diluted, used directly)
8% myrrh gum
0.6% natural essences of aromatic plants such as peppermint, mint, thyme, rosemary and lavender
1% stevia
0.4% vegetable dye

Myrrh gum gives the end product a broad spectrum antiseptic and anti-inflammatory properties. The brine of the present invention provides a wide range of minerals that nourish tissues and favor cell regeneration, stimulates the immune system and has certain disinfecting power. Its alkalinity controls oral acidity occurring after meals, reducing the risk of wearing off the enamel and the risk of cavities.

The method of manufacture is simple and consists of dissolving all the ingredients in the brine of the present invention, forming a stable and uniform solution. (not in the scope of the claims)

Sunscreen prepared with the brine of the present invention is natural, and its sun-screening action is based on physical, not chemical, barriers. It contains zinc oxide and titanium dioxide, natural components that do not penetrate the skin and act by forming "a screen consisting of small mirrors" that reflect solar rays, being effective for type A and B UV rays.

Argan oil, another ingredient in the formula, softens, hydrates and protects skin against dryness. A Tween 20 type solubilizer is added to dissolve this oil in the brine of the present invention.

The brine of the present invention provides minerals to the product, nourishing the cells, hydrating them and favoring healing and self-repair. It has detoxifying and immunostimulatory power.

The ingredients of this formula are:
88% brine of the present invention (not diluted, used directly)
8% natural argan oil
0.5% zinc oxide
0.5% titanium dioxide
2% Tween 20
1% natural aromas

### Example 6: Nasal spray (not in the scope of the claims)

The nasal spray of the present invention contains the non-diluted brine of the present invention, i.e., it is a hypertonic product with a salt concentration of 3.3%, about 33 g/l of minerals.

The antiseptic, anti-inflammatory and decongestant properties of seawater when it is applied to the nasal passages, particularly in cases of colds and allergies, are well known.

The nasal spray product of the present invention containing the brine is sterilized and free of any biological and chemical pollutant, so it is suitable for said use. The presentation of the product is a nebulizer with an ergonomic applicator suitable for the nasal passages for the correct and comfortable application thereof by the end user.

### Example 7: Animal feed

The animal feeds contain common salt (NaCl) at a concentration of about 0.51%.

In this case, the brine of the present invention incorporated in the product replaces this common salt in addition to many other rock minerals added in an artificial manner. The over 90 minerals provided by the brine of the present invention are much more readily assimilable by livestock, and they give the feed excellent nutritional value. They stimulate the immune system as well as the gastrointestinal and renal systems, making them more resistant to contracting diseases.

The feed is presented in the form of pellets and contains the following ingredients:
40% micronized soy
10% concentrated cheese whey protein
1% lysine, methionine, choline
3% milk powder
20% extruded soybean
20% extruded corn
1.5% lactose
0.5% vitamins A, D, E, B1, B2, B6, B12, K3
0.5% folic acid
0.5% nicotinic acid
1% antioxidants
2% brine of the present invention without being previously diluted.

### Example 8: Natural mineral supplement for stock raising

The brine of the present invention can be used in liquid form as a mineral supplement for correct nutrition, stimulating the metabolism of animals and protecting them against contracting diseases. To that end, the non-diluted brine of the present invention is mixed with the animals' usual drinking water at a proportion of 28% by weight.

Mixing the brine of the present invention in this proportion with fresh water from the general supply obtains an isotonic mineral solution, i.e., the solution has a concentration of minerals of about 9 g/l. This is the concentration of salts in the animals' blood and the intra- and extracellular water.

The product has already been administered to cows, pigs, sheep and horses with very good results. The rate of illness decreases by more than 90%. Its effect on preventing diseases related to the lung and the gastrointestinal system is particularly notable. Its effect on growth and weight gain of the animals is also noted.

The method for obtaining this product is very simple and involves mixing 2 parts of the brine of the present invention in 5 parts of mains water.

## Claims

1. A brine obtainable from seawater, **characterized in that** it presents the mineral composition of the seawater in its natural state, with a boron content less than 1 mg/l, free of organic components, bacteria and macromolecules of a size greater than 0.1 µ, a pH between 8.6 - 8.8, is vitalized, and presents the angle in the molecular structure of the bonds of the hydrogen atoms with the oxygen atom of highly pure natural spring water.

2. A method for obtaining a brine according to claim 1, comprising the following steps:
a) Seawater extraction,
b) Purity analysis,
c) First microfiltration,
d) Deboronation treatment,
e) Increase in pH,
f) Vitalization treatment,
g) Second microfiltration,
h) Microbiological analysis,
i) Packaging.

3. The method according to claim 2, **characterized in that** the microfiltration step c) is performed with membrane filters with a pore size of 1 µ and 0.22 µ.

4. The method according to claim 2 or 3 , **characterized in that** the deboronation step d) is performed with a boron-selective ion exchange demineralization system.

5. The method according to claim 2, 3, or 4, **characterized in that** a mixture of bicarbonate/carbonate is added at a ratio of 5:1 in the increase in pH step e).

6. The method according to any of claims 2 to 5, **characterized in that** the microfiltration step g) is performed with membrane filters with a pore size of 0.1 µ.

7. Use of the brine according to claim 1 for the preservation and treatment of fish and shellfish.

8. Use of the brine according to claim 1 as a condiment.

## Patentansprüche

1. Salzlake, die aus Meerwasser erhalten werden kann, **dadurch gekennzeichnet, dass** sie die Mineralzusammensetzung des Meerwassers in seinem natürlichen Zustand aufweist, mit einem Borgehalt niedriger als 1 mg/l, frei von organischen Bestandteilen, Bakterien und Makromolekülen mit einer Größe von mehr als 0,1 µ, mit einem pH-Wert zwischen 8,6 und 8,8, dass sie belebt ist und den Winkel in der Molekülstruktur der Bindungen der Wasserstoffatome mit dem Sauerstoffatom des hochreinen, natürlichen Quellwassers aufweist.

2. Verfahren zum Erhalten von einer Salzlake nach Anspruch 1, die folgenden Schritte umfassend:
a) Entnahme von Meerwasser
b) Reinheitsanalyse
c) erste Mikrofiltration
d) Deborierungsbehandlung
e) Erhöhung des pH-Werts
f) Behandlung zur Belebung
g) zweite Mikrofiltration
h) mikrobiologische Analyse
i) Verpackung

3. Verfahren nach Anspruch 2, **dadurch gekennzeichnet, dass** der Mikrofiltrationsschritt c) mit Membranfiltern mit einer Porengröße von 1 µ und 0,22 µ durchgeführt wird.

4. Verfahren nach Anspruch 2 oder 3, **dadurch gekennzeichnet, dass** der Deborierungsschritt d) mit einem Bor-selektiven System zur Demineralisierung durch Ionenaustausch durchgeführt wird.

5. Verfahren nach Anspruch 2, 3 oder 4, **dadurch gekennzeichnet, dass** in Schritt e) zur Erhöhung des pH-Werts ein Gemisch aus Bicarbonat/Carbonat in einem Verhältnis von 5:1 zugegeben wird.

6. Verfahren nach einem von Anspruch 2 bis 5, **dadurch gekennzeichnet, dass** der Mikrofiltrationsschritt g) mit Membranfiltern mit einer Porengröße von 0,1 µ durchgeführt wird.

7. Verwendung der Salzlake nach Anspruch 1 zur Konservierung und Behandlung von Fisch und Meeresfrüchten.

8. Verwendung der Salzlake nach Anspruch 1 als Würzmittel.

## Revendications

1. Saumure pouvant être obtenue à partir d'eau de mer, **caractérisée en ce qu'**elle présente la composition minérale de l'eau de mer dans son état naturel, avec une teneur en bore inférieure à 1 mg/l., exempte de composants organiques, de bactéries et de macromolécules d'une taille supérieure à 0,1 µ, un pH entre 8.6 - 8.8, est vitalisée, et présente l'angle dans la structure moléculaire des liaisons des atomes d'hydrogène avec l'atome d'oxygène d'eau de source naturelle très pure.

2. Procédé d'obtention d'une saumure, selon la revendication 1, comprenant les étapes suivantes :
a) L'extraction de l'eau de mer,
b) L'analyse de la pureté,
c) La première microfiltration,
d) Le traitement de déboronisation,
e) L'augmentation du pH,
f) Le traitement de vitalisation,
g) La deuxième microfiltration,
h) L'analyse microbiologique,
i) L'emballage.

3. Procédé selon la revendication 2, **caractérisé en ce que** l'étape de microfiltration c) est réalisée avec des filtres à membrane avec une taille de pores de 1 µ et 0,22 µ.

4. Procédé selon la revendication 2 ou 3, **caractérisé en ce que** l'étape de déboronisation d) est réalisée avec un système de déminéralisation par échange d'ions sélectif pour le bore.

5. Procédé selon la revendication 2, 3, ou 4, **caractérisé en ce qu'**un mélange de bicarbonate/carbonate est ajouté à un rapport de 5:1 dans l'augmentation du pH de l'étape e).

6. Procédé selon l'une quelconque des revendications 2 à 5, **caractérisé en ce que** l'étape de microfiltration g) est réalisée avec des filtres à membrane avec une taille de pores de 0,1 µ.

7. Utilisation de la saumure selon la revendication 1, pour la conservation et le traitement de poissons et crustacés.

8. Utilisation de la saumure selon la revendication 1 comme condiment.
